# EUROPEAN PATENT APPLICATION

(11) **EP 4 649 919 A1**
(43) Date of publication of application: **19.11.2025**
(21) Application number: 24020148.3
(22) Date of filing: 13.05.2024
(51) Int. Cl.: A61F 2/24

(54) **IMPLANT FOR IMPROVING COAPTATION OF AN ATRIOVENTRICULAR VALVE IN A HUMAN HEART**

(71) Applicant: AVVie GmbH, 1090 Vienna (AT)
(72) Inventor: MOHL, Werner, 2571 Altenmarkt/Thennenberg (AT); KHIENWAD, Thananya, 1190 Wien (AT); GRADISCHAR, Andreas, 1170 Wien (AT); YOHANAN, Ziv, 1140 Wien (AT)
(74) Representative: SONN Patentanwälte GmbH & Co KG

(57) **Abstract**

An implant (1) for improving coaptation of an atrioventricular valve in a human heart, comprising a frame that comprises a lower frame section (2) that supports an artificial leaflet (4), the artificial leaflet (4) being arranged and configured to cover the first native leaflet and forms a coaptation plane with a free edge for coaptation with the second native leaflet, wherein the lower frame section (2) comprises or forms at least one central clasp (5) for attaching the lower frame section (2) to the first native leaflet, the frame further comprising an upper frame section (3) that supports a flexible sealing skirt (6) made of a two-dimensional element curved to rest against the atrial wall along the posterior annulus section, wherein the upper frame section (3) is made of a resilient material so that free end regions (7) of the upper frame section (3) are capable of being resiliently braced against the atrial wall and/or against the posterior annulus section.

## Description

The invention refers to an implant for improving coaptation of an atrioventricular valve in a human heart, in particular a mitral valve, the atrioventricular valve having a first and a second native leaflet, an anterolateral and posteromedial commissure between the first and the second native leaflet, and an annulus adjacent an atrial wall of the heart comprising a posterior annulus section and an anterior annulus section.

The mitral valve is one of the four heart valves that regulate blood flow through the heart. It is located between the left atrium and the left ventricle and consists of two leaflets, referred to as the anterior and posterior leaflets. Proper functioning of the mitral valve depends on the coordinated interaction of its various components, including the leaflets, chordae tendineae, papillary muscles, and the annulus.

Mitral valve regurgitation is a common valvular heart disease characterized by the improper closure of the mitral valve leaflets, leading to the backward flow of blood from the left ventricle into the left atrium. This can result in reduced cardiac output and increased workload on the heart, potentially causing symptoms such as fatigue, shortness of breath, and fluid retention.

Various surgical techniques have been developed to repair or replace the mitral valve in patients with mitral regurgitation.

Mitral valve repair techniques aim to restore the normal function of the native valve by correcting the underlying anatomical defects. These techniques include annuloplasty, which involves the implantation of a ring or band to reshape and stabilize the mitral annulus; leaflet resection, which involves the removal of excess or diseased leaflet tissue; and chordal manipulation, which involves the shortening, transposition, or replacement of the chordae tendineae.

Mitral valve replacement involves the removal of the native valve and its replacement with a prosthetic valve. Prosthetic valves can be either mechanical or bioprosthetic. Mechanical valves are made of synthetic materials and require lifelong anticoagulation therapy to prevent thromboembolism. Bioprosthetic valves, on the other hand, are made of biological tissue and do not require anticoagulation, but they have a limited durability compared to mechanical valves.

Another promising approach to treating mitral valve regurgitation involves the use of transcatheter mitral valve repair (TMVR) devices that incorporate an artificial leaflet supported on a foldable frame structure. These devices are designed to be inserted into the native mitral valve and attached to the annulus and the dysfunctional native leaflet, allowing the artificial leaflet to cover the dysfunctional leaflet and form a coaptation plane for coaptation with the opposite native leaflet.

The use of TMVR devices with artificial leaflets has several potential advantages over other mitral valve repair and replacement techniques. First, these devices can be delivered percutaneously, which minimizes the invasiveness of the procedure and reduces the risks associated with open-heart surgery. Second, by preserving the native valve apparatus and only replacing the dysfunctional leaflet, these devices may help to maintain the natural geometry and function of the mitral valve.

The deployment and attachment of TMVR devices involve a series of steps that are designed to ensure the proper positioning and functioning of the device within the native mitral valve.

The first step in the deployment process is the percutaneous access and delivery of the device to the mitral valve. This is typically achieved via a transseptal, transatrial or transapical approach, depending on the specific device and patient anatomy. The transseptal approach involves the creation of a small puncture in the interatrial septum, allowing the device to be advanced from the right atrium into the left atrium and then into the mitral valve. The transapical approach, on the other hand, involves the creation of a small incision in the apex of the left ventricle, allowing the device to be advanced directly into the mitral valve. The transatrial approach allows the surgical deployment by minimal invasive transthoracic surgery.

Once the device has been advanced to the mitral valve, the next step is the positioning and deployment of the device within the valve. This is typically achieved using fluoroscopic and echocardiographic guidance to ensure the proper alignment of the device with the native valve anatomy. The self-expanding frame of the device is then released from the delivery catheter, allowing it to expand and conform to the shape of the mitral annulus and the dysfunctional native leaflet.

The attachment of the device to the native valve involves two main mechanisms: annular anchoring and leaflet attachment. Annular anchoring is typically achieved through the use of a series of radial force-exerting elements or barbs on the frame of the device that engage the mitral annulus and prevent device migration or embolization. Leaflet attachment, on the other hand, is achieved through the use of a series of grasping elements, clasps or tabs on the frame of the device that engage the dysfunctional native leaflet and hold it in place.

Once the device has been deployed and attached to the native valve, the artificial leaflet of the device begins to function as a new coaptation surface for the opposite native leaflet. During systole, the artificial leaflet and the opposite native leaflet coapt, meaning that they come together to close the mitral orifice and prevent the regurgitation of blood from the left ventricle into the left atrium. During diastole, the artificial leaflet and the opposite native leaflet open to allow the unobstructed flow of blood from the left atrium into the left ventricle.

The artificial leaflet of TMVR devices is typically made of a flexible, biocompatible material, such as pericardial tissue or a polymeric membrane, that is designed to mimic the natural motion and function of the native leaflet. The material is also designed to be durable and resistant to calcification and degeneration over time.

One of the significant challenges associated with TMVR devices, including those with artificial leaflets, is the occurrence of paravalvular leakage (PVL). PVL refers to the leakage of blood around the perimeter of the device, between the device frame and the native valve annulus or leaflets. This leakage can result in residual mitral regurgitation, which can reduce the effectiveness of the device and lead to suboptimal clinical outcomes.

There are several factors that can contribute to the occurrence of PVL in TMVR devices. One of the main factors is the incomplete or inadequate sealing of the device frame against the native valve annulus. This can occur due to the irregular or calcified nature of the annulus, which can prevent the device from achieving a uniform and circumferential seal. Additionally, the presence of significant annular dilatation or distortion can create gaps or spaces between the device frame and the annulus, allowing blood to leak through.

Another factor that can contribute to PVL is the incomplete or inadequate attachment of the device to the dysfunctional native leaflet. If the grasping elements or tabs on the device frame fail to adequately engage and secure the leaflet, it can result in a gap or space between the artificial leaflet and the native leaflet, allowing blood to leak through.

The occurrence of PVL can also be influenced by the design and sizing of the TMVR device. Devices with a larger profile or a more rigid frame may be more prone to PVL, as they may not be able to conform as well to the native valve anatomy. Similarly, devices that are undersized or oversized relative to the native valve may be more prone to PVL, as they may not be able to achieve a proper seal or attachment.

The implant of the present invention aims to overcome the problem of paravalvular leakage (PVL) associated with transcatheter mitral valve repair (TMVR) devices, namely those that utilize an artificial leaflet.

In order to solve this and other objects, the invention provides an implant comprising a frame that comprises a lower frame section that supports an artificial leaflet, the artificial leaflet being arranged and configured to cover the first native leaflet and forms a coaptation plane for coaptation with the second native leaflet, wherein the lower frame section comprises or forms at least one central clasp for attaching the lower frame section to the first native leaflet, the frame further comprising an upper frame section that supports a flexible sealing skirt made of a two-dimensional element curved to rest against the atrial wall along the posterior annulus section, wherein the upper frame section is made of a resilient material so that free end regions of the upper frame section are capable of being resiliently braced against the atrial wall and/or against the posterior annulus section.

According to the invention, a secure and effective seal against the native valve annulus, particularly along the posterior annulus section, is achieved through the use of a flexible sealing skirt that is supported by the upper frame section of the device. The sealing skirt preferably creates a dome for collecting blood so as to prevent a paravalvular route to the atrium. The sealing skirt is made of a two-dimensional element that is curved to rest against the atrial wall along the posterior annulus section. This curved configuration allows the sealing skirt to conform to the contours of the annulus and to create a tight, circumferential seal around the perimeter of the device. The flexibility of the sealing skirt also allows it to adapt to the irregular or calcified surface of the annulus, further enhancing its sealing properties.

The use of a resilient material for the upper frame section of the implant allows the free end regions of the upper frame section to be resiliently braced against the atrial wall and/or against the posterior annulus section. This resilient bracing helps to maintain the proper positioning and orientation of the sealing skirt and the artificial leaflet, even in the presence of dynamic changes in the anatomy of the mitral valve during the cardiac cycle. The resilient material also allows the upper frame section to adapt to the individual anatomy of each patient, providing a more personalized and conformable fit.

Another technical effect of the implant is its ability to provide a stable and durable attachment to the dysfunctional native leaflet. This is achieved through the use of a central clasp that is formed or supported by the lower frame section of the device. The central clasp is designed to grasp and secure the free edge of the dysfunctional native leaflet, creating a robust and reliable connection between the artificial leaflet and the native leaflet. This connection helps to ensure that the artificial leaflet remains properly positioned and oriented throughout the cardiac cycle, and that it can withstand the high pressures and forces generated during systole.

The combination of features of the implant of the invention reduces the risk of paravalvular leakage and ensures the proper functioning of the artificial leaflet. Furthermore, the technical effects of the implant also contribute to its ease of use and deliverability. The self-expanding nature of the frame and the flexibility of the sealing skirt allow the implant to be compressed into a low-profile configuration for delivery through a catheter-based system. Once deployed, the implant can expand and conform to the native anatomy without the need for complex or time-consuming adjustments.

The invention refers to a hemi valve concept, the implant being deployed by sliding the same over the native posterior leaflet, spreading the free end regions and stabilizing the implant with passive anchoring in the annular region.

The frame of the implant can be made from a variety of biocompatible materials that provide the necessary strength, flexibility, and durability for the intended application. The frame can be formed from one or more wires, with the wires being shaped to create the desired geometry and mechanical properties. Alternatively, the frame may be manufactured by cutting it from a nitinol plane sheet and thermoforming the structures to force the implant into the anulus. One particularly suitable material for the frame is a superelastic or shape memory alloy, such as Nitinol (NiTi). Nitinol is a well-known and widely used material in the field of medical devices, particularly for self-expanding and resilient structures. The frame can be formed from one or more wires made of Nitinol. The Nitinol wire can be woven, braided, or laser-cut to create the desired pattern and configuration of the frame, with the lower frame section supporting the artificial leaflet and the central clasp, and the upper frame section supporting the sealing skirt. The diameter and cross-sectional shape of the Nitinol wire or the dimensions of the laser cut Nitinol sections of the frame can be varied to optimize the strength, flexibility, and radial force of the frame, depending on the specific requirements of the implant and the patient's anatomy.

The artificial leaflet and the sealing skirt of the implant can be made from a variety of biocompatible materials that provide the necessary flexibility, durability, and tissue compatibility for the intended application. One suitable material is bovine or porcine pericardial tissue. Pericardial tissue can be cut, shaped, and sutured to create the desired geometry and size of the artificial leaflet and the sealing skirt, respectively, and can be mounted onto the lower frame section and the upper frame section, respectively, of the implant using various techniques, such as suturing or adhesive bonding.

Another suitable material for the artificial leaflet and/or the sealing skirt is a synthetic or polymeric material, such as polytetrafluoroethylene (PTFE), polyester, or polyurethane. These materials can be fabricated into thin, flexible sheets or membranes that can be cut, shaped, and attached to the frame using similar techniques as for pericardial tissue.

In a preferred embodiment of the invention, the upper frame section comprises an annulus bar that extends in a curved manner along the posterior annulus section, wherein the opposite free end regions of the annulus bar are capable of being resiliently braced against the posterior annulus section and/or the atrial wall. In this way, the annulus bar is able to create a stable and secure anchoring of the implant along the posterior annulus section. The curved configuration of the annulus bar allows it to conform to the natural shape and contours of the posterior annulus. By matching the geometry of the annulus bar to the anatomy of the posterior annulus, the implant can achieve a more precise and customized fit, reducing the risk of gaps, leaks, or instability. Further, the annulus bar provides a resilient bracing force against the posterior annulus and/or the atrial wall. The opposite free end regions of the annulus bar are designed to be resiliently deformable, allowing them to flex and adapt to the dynamic changes in the anatomy of the mitral valve (in the anterior aspect of the atrial wall and arising from the anterior leaflet) during the cardiac cycle. As the annulus and the atrial wall move and deform during systole and diastole, the free end regions of the annulus bar can maintain a constant and gentle pressure against the tissue, ensuring a stable and leak-free interface.

The resilient bracing force provided by the annulus bar also helps to distribute the mechanical loads and stresses more evenly along the posterior annulus, reducing the risk of localized stress concentrations or tissue damage. This is particularly important in patients with calcified or fragile annular tissue, where excessive or uneven forces could lead to annular rupture or dehiscence of the implant.

In addition to its anchoring and bracing functions, the annulus bar also provides a support structure for the sealing skirt of the implant. The lower edge region of the sealing skirt can be attached to the annulus bar along its length, creating a continuous and seamless interface between the implant and the atrial wall.

The annulus bar can be made from the same or similar materials as the rest of the frame, such as Nitinol wire, and can be fabricated using various techniques, such as shape setting, laser cutting, or wire forming. The cross-sectional shape and diameter of the annulus bar can be optimized to provide the desired mechanical properties, such as flexibility, stiffness, and strength.

In another preferred embodiment of the invention, the upper frame section comprises an atrial bar that extends in a curved manner along the atrial wall adjacent the posterior annulus section, wherein the opposite free end regions of the atrial bar are capable of being resiliently braced against the atrial wall. By extending the upper frame section beyond the posterior annulus and into the atrial cavity, the atrial bar can engage a larger surface area of the atrial wall, providing a more robust and redundant anchoring mechanism.

The curved configuration of the atrial bar allows it to conform to the natural shape and curvature of the atrial wall, which can vary significantly among patients depending on factors such as age, anatomy, and disease state.

As for the annulus bar, the atrial bar provides a resilient bracing force against the atrial wall. Similar to the annulus bar, the opposite free end regions of the atrial bar are designed to be resiliently deformable, allowing them to flex and adapt to the dynamic changes in the shape and position of the atrial wall during the cardiac cycle.

In addition to its anchoring and bracing functions, the atrial bar also provides a support structure for the sealing skirt of the implant. The upper edge region of the sealing skirt can be attached to the atrial bar along its length, creating a wider and more compliant sealing zone that can accommodate the variable anatomy and motion of the atrial wall. The atrial bar can be made from the same or similar materials as the rest of the frame, such as Nitinol wire, and can be fabricated using the same or similar techniques, such as shape setting, laser cutting, or wire forming.

In a preferred embodiment of the invention, the free end regions comprise an undulating section, the undulating section preferably connecting an attachment structure, such as an eyelet, located at a distal section of the free end regions with a proximal section thereof. One of the primary technical effects of the undulating section is its ability to enhance the flexibility and conformability of the free end regions of the annulus bar and/or the atrial bar. The undulating shape of this section allows it to act as a spring-like element that can absorb and distribute the mechanical forces and deformations imposed by the cardiac cycle. As the annulus and the atrial wall move and change shape during systole and diastole, the undulating section can flex and bend accordingly, maintaining a constant and gentle contact with the tissue.

The enhanced flexibility and conformability provided by the undulating section also help to reduce the risk of tissue damage or irritation that could result from excessive or concentrated forces. By allowing the free end regions to adapt to the dynamic anatomy of the annulus and the atrial wall, the undulating section can minimize the stresses and strains imposed on the tissue, promoting a more biocompatible and atraumatic interface.

The undulating section can be formed as an integral part of the annulus bar and/or the atrial bar, using the same or similar materials and fabrication techniques. The geometry and dimensions of the undulating section, such as the number and amplitude of the undulations, can be optimized to provide the desired mechanical properties and performance characteristics.

The attachment structure, such as an eyelet, is typically located at the distal tip or end of the free end regions, and is designed to provide a means for anchoring or securing the implant to the surrounding tissue or anatomical structures. In the case of the annulus bar, the eyelet may be used to anchor the implant to the posterior annulus or the adjacent atrial wall, while in the case of the atrial bar, the eyelet may be used to anchor the implant to the atrial wall or other suitable locations.

The eyelets also function to press the sealing skirt into the atrial wall, thus forming a dome to prevent PVL.

In a preferred embodiment of the invention, the free end regions of the annulus bar and the free end regions of the atrial bar are connected with each other by means of a connecting wire, which preferably has an undulating shape. This connecting wire is provided to enhance the structural integrity and stability of the upper frame section of the implant. By linking the free end regions of the annulus bar and the atrial bar, the connecting wire creates a continuous framework that can better withstand the mechanical forces and deformations imposed by the cardiac cycle. This is useful in preventing a dislocation of the annulus bar and the atrial bar, which could compromise the anchoring, sealing, and functioning of the implant. By acting as a bridge or a tie between the free end regions of the annulus bar and the atrial bar, the connecting wire can help to balance and equalize the forces exerted by these components on the surrounding tissue.

The undulating shape allows the connecting wire to act as a spring-like element that can absorb and dissipate the mechanical energy generated by the cardiac cycle. Further, the undulating shape of the connecting wire allows it to adapt to the dynamic changes in the anatomy and position of the annulus and the atrial wall, without imposing excessive or rigid constraints on the movement of the upper frame section. As the annulus and the atrial wall expand and contract during the cardiac cycle, the undulating shape of the connecting wire can flex and deform accordingly, maintaining a constant and gentle contact with the tissue.

According to a preferred embodiment, the free end regions of the annulus bar have a greater flexural rigidity in a first bending direction than in a second bending direction that extends orthogonal to the first bending direction, wherein limiting bending in the first bending direction is for preventing the implant from falling into the ventricle and bending in the second bending direction is for resiliently bracing the free end regions against the posterior annulus section and/or the atrial wall. Preferably, the flexural rigidity (indicated in N.m²) in the first bending direction is at least twice the flexural rigidity in the second bending direction. The flexural rigidity, also referred to as bending stiffness, of the free end regions of the annulus bar may preferably be designed by adjusting the second moment of area of the cross section of the free end regions. For example, the cross section of the free end regions may be designed to be rectangular with a dimension of the rectangle in the first direction being larger than the dimension in the second direction, thereby providing a greater second moment of area in the first bending direction than in the second bending direction.

The greater bending stiffness in the first direction is preferably provided over a length of the annulus bar extending from the commissure to the undulating section of the free end regions.

According to another preferred embodiment, a plurality of resilient spring elements are extending form the annulus bar and/or from the atrial bar for pressing the sealing skirt against the atrial wall. In this way, the resilient spring elements extend into the space between the annulus bar and the atrial bar, where they can create a more direct sealing force that is specifically targeted at the interface between the sealing skirt and the atrial wall. Preferably, the spring elements are extending from the atrial bar only.

In a preferred embodiment of the invention, the upper frame section carries a cover sheet that, together with the sealing skirt, forms a pocket for receiving retrograde blood flow. This configuration enhances the sealing performance of the prosthetic valve. In particular, a dedicated space or pocket is provided between the cover sheet and the sealing skirt, which can capture and contain the retrograde blood flow that may occur during systole. By providing a pocket for receiving this retrograde blood flow, the implant can effectively prevent or minimize the amount of blood that leaks back into the atrium. The pocket design allows the retrograde blood flow to exert a positive pressure or force on the sealing skirt, which increases the contact and conformability of the skirt against the atrial wall.

This self-adjusting sealing mechanism is particularly advantageous for accommodating the dynamic changes in the anatomy and the hemodynamics of the heart throughout the cardiac cycle. As the pressure and the volume of the regurgitant blood vary during systole and diastole, the pocket can expand or contract accordingly, maintaining a constant and optimal sealing force on the skirt.

The pocket preferably extends along the entire with of the upper frame section, i.e. along entire length of the posterior annulus.

The cover sheet is preferably fixed to both, the annulus bar and the atrial bar. The same is the case for the sealing skirt, wherein the sealing skirt is dimensioned such that it can bulge towards the annulus and/or the atrial wall.

The cover sheet preferably forms the outer or atrial-facing surface of the pocket, whereas the sealing skirt forms the inner or ventricular-facing surface of the pocket.

The spring elements supported on the atrial bar may be arranged to extend into the pocket and to exert pressure onto the sealing skirt from the inside of the pocket.

In a preferred embodiment of the invention, the sealing skirt comprises one or more openings for allowing retrograde blood flow to enter into the pocket. This facilitates retrograde blood flow into the pocket formed between the cover sheet and the sealing skirt. Preferably, a plurality of openings are distributed along the circumferential direction. The openings are preferably arranged adjacent to the annulus bar.

In a preferred embodiment of the invention, the central clasp is connected to, or integrally formed with, a central section of the annulus bar arranged between the opposite free end regions of the annulus bar. This results in the creation of a direct load-bearing pathway between the clasp and the upper frame section of the implant.

The central clasp is preferably formed from a single piece of bent wire and resembles the rounded shape of a capital letter "W" with four curved segments connected by radii. The clasp is designed to grasp the native leaflet. The two outer curved segments of the "W" shape together function as one leg of the clasp positioned on one side of the leaflet. The two inner curved segments of the "W" shape, which are the segments that connect to form the middle peak of the letter, form the second leg of the clasp. This second leg is arranged on the opposite side of the leaflet compared to the first leg. Thus, when the central clasp grasps the native leaflet, the leaflet is positioned and clamped between the outer two curved segments and the inner two segments. In other words, the clasp forms a pocket between the outer two curved segments and the inner two segments for receiving the native leaflet.

In a preferred embodiment of the invention, the lower frame section comprises an anterolateral commissural clasp for clasping the lower frame section to the anterolateral commissure and a posteromedial commissural clasp for clasping the lower frame section to the posteromedial commissure, the central clasp being arranged between the anterolateral commissural clasp and the posteromedial commissural clasp.

Incorporating the anterolateral and posteromedial commissural clasps into the lower frame section enhances the anchoring and fixation of the implant to the native valve anatomy. The commissures are the points where the anterior and posterior leaflets of the mitral valve meet and are attached to the annulus and the surrounding myocardium. By clasping the lower frame section to these commissures, the implant can achieve a more secure and anatomically correct positioning within the native valve, which can help to prevent device migration, rotation, or dislodgement.

The commissural clasps and the central clasp can be fabricated from the same or similar materials as the rest of the frame, such as Nitinol or other shape memory alloys, and can be formed using various techniques such as laser cutting, shape setting, or wire forming.

Preferably, the commissural clasps comprise two essentially parallel legs arranged opposite each other and connected by a curved frame section for clamping the anterolateral and the posteromedial commissure, respectively, between said two legs. Therein, the curved frame section connecting the two legs embraces the edge or rim of the native leaflet.

According to a preferred embodiment of the invention, the anterolateral commissural clasp and the posteromedial commissural clasp are connected to, or integrally formed with, the annulus bar, preferably the free end regions of the annulus bar. By directly linking the commissural clasps to the annulus bar, which is the main anchoring component of the upper frame section, the implant can achieve a more robust and efficient transfer of the forces and stresses between the native valve and the prosthetic valve. The commissural clasps can be connected to the annulus bar through various means, such as welding, soldering, crimping, or other suitable methods, depending on the specific materials and design of the components. In some cases, the commissural clasps and the annulus bar can be integrally formed as a single component.

In a preferred embodiment of the invention, the lower frame section of the implant comprises leaflet support bars, to which the artificial leaflet is attached. These leaflet support bars preferably extend in a ventricular direction from the annulus bar. Additionally, a first one of said leaflet support bars may be arranged between the central clasp and the anterolateral commissural clasp, while a second one of said leaflet support bars may be arranged between the central clasp and the posteromedial commissural clasp. One effect of incorporating leaflet support bars into the lower frame section is the provision of a stable and reliable foundation for the attachment and support of the artificial leaflet. By attaching the leaflet to the support bars, the implant can ensure that the leaflet maintains its proper shape, position, and mobility throughout the cardiac cycle.

Moreover, the attachment points, at which the leaflet is attached to the leaflet support bars divide the leaflet into three sections, corresponding to the P1, P2, and P3 scallops of a native posterior leaflet. In the native mitral valve, the posterior leaflet is typically divided into three scallops, named P1, P2, and P3, based on their relative positions and morphological characteristics. The P1 segment corresponds to the anterolateral portion of the leaflet, the P2 segment corresponds to the middle portion, and the P3 segment corresponds to the posteromedial portion. Each segment has a unique shape, size, and mobility that contributes to the overall coaptation and sealing of the valve. By designing the artificial leaflet to mimic this natural segmentation pattern, the implant can achieve a more physiologic and conformable leaflet geometry that better adapts to the native valve anatomy. This can help to optimize the coaptation and sealing of the leaflet against the native anterior leaflet, reducing the risk of paravalvular leakage or central regurgitation.

The leaflet support bars can be fabricated from the same or similar materials as the rest of the frame, such as Nitinol or other shape memory alloys, and can be formed using various techniques such as laser cutting, shape setting, or wire forming.

Preferably, the artificial leaflet comprises an upper edge attached to the annulus bar and a lower edge, opposite the upper edge, that comprises a plurality of attachment points arranged at a distance from each other, wherein the lower edge, at said attachment points, is fixed to the leaflet support bars, the anterolateral commissural clasp and the posteromedial commissural clasp. Preferably, a lower edge of the artificial leaflet is attached to the lower frame section at said plurality of attachment points only. Thus, there are no additional frame elements for holding the artificial leaflet at its lower edge. Therefore, the lower edge of the artificial leaflet, in the region between two attachments points can freely move and adapt its shape, thereby forming a "free" edge.

In a preferred embodiment of the invention, the leaflet support bars are connected to each other, preferably at their free ends, by means of a cross bar. Connecting the leaflet support bars to each other by means of a cross bar results in the creation of a closed and self-supporting frame structure within the lower frame section of the implant. By linking the free ends of the support bars, which are typically the most distal and flexible portions of these components, the cross bar can provide a stabilizing and reinforcing effect that helps to maintain the shape, position, and alignment of the support bars throughout the cardiac cycle.

According to another preferred embodiment, the opposite end regions of the annulus bar are each split into an upper arm and a lower arm, wherein preferably the upper arms are capable of being resiliently braced against the posterior annulus section and wherein preferably the anterolateral commissural clasp and the posteromedial commissural clasp and preferably the leaflet support bars are connected to, or integrally formed with lower arms.

The technical effect of splitting the end regions of the annulus bar into upper and lower arms is the ability to individually adapt these components to the specific mechanical requirements and anatomical constraints of the upper and lower frame sections, respectively. By separating the arms, the implant can optimize the design and material properties of each arm to better suit its intended function and location within the native valve anatomy.

The upper arms, which are part of the upper frame section, are preferably capable of being resiliently braced against the atrial wall. This means that they can be designed with a higher degree of flexibility and conformability compared to the lower arms, allowing them to better adapt to the shape and movement of the atrial wall. The resilient bracing effect of the upper arms is particularly important for maintaining a stable and secure anchoring of the implant within the annulus, preventing migration or dislodgment of the prosthetic valve over time.

On the other hand, the lower arms, which are part of the lower frame section, are preferably connected to or integrally formed with the anterolateral commissural clasp, the posteromedial commissural clasp, and the leaflet support bars. This configuration allows the lower arms to serve as a rigid and stable foundation for the artificial leaflet and its supporting structures, ensuring their proper position and function throughout the cardiac cycle. To fulfill this role, the lower arms can be designed with a higher degree of stiffness and strength compared to the upper arms, providing a more robust and load-bearing framework for the leaflet and clasps. This can be achieved by using a thicker cross-section, a more rigid material composition, or a more reinforced geometric shape compared to the upper arms.

Moreover, the lower arms can be adapted to follow the curvature of the annulus, providing a more anatomically correct and conformable support for the leaflet and clasps.

Whereas the bars have an increased stiffness and stabilize the implant, especially over the anterior leaflet, their endings are constructed flexible to prevent trauma to the atrial wall.

The attachment points, where the artificial leaflet or the sealing skirt is attached to the frame, can be designed in various embodiments to ensure a secure, durable, and reliable connection between these components. One embodiment is the use of loops or eyelets, which are small, circular or oval-shaped openings or protrusions that are integrated into the frame structure. These loops or eyelets can be provided along the annulus bar, the leaflet support bars, or the commissural clasps. The leaflet or skirt can then be sutured, tied, or otherwise fastened to these loops or eyelets. Other possible embodiments of the attachment points include hooks, barbs, or anchors, which can penetrate or grasp the leaflet or skirt material. Additionally, the attachment points can be designed as slots, grooves, or channels, which can accommodate the edge or the surface of the leaflet or skirt.

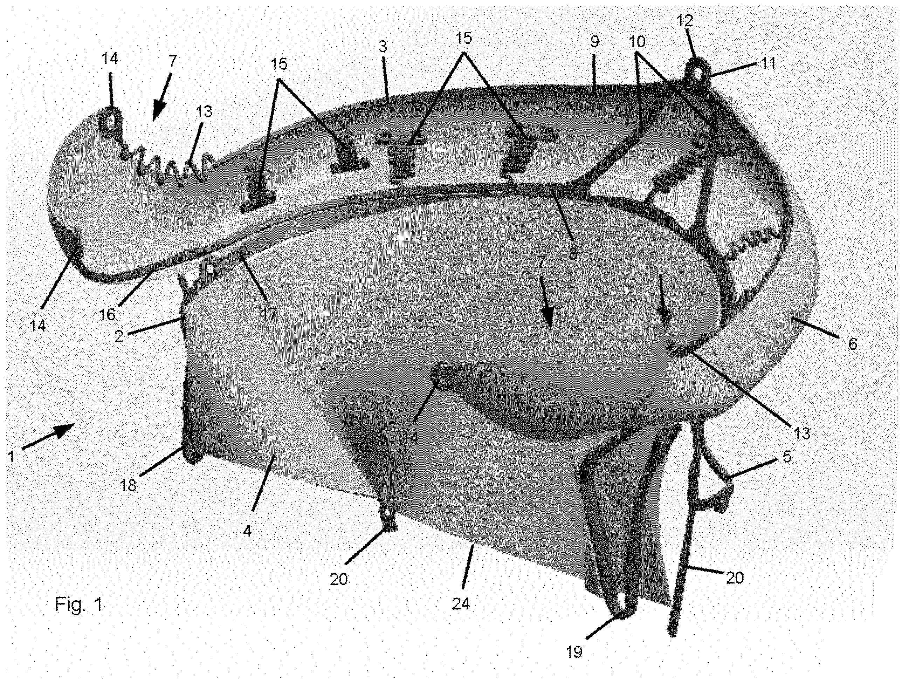

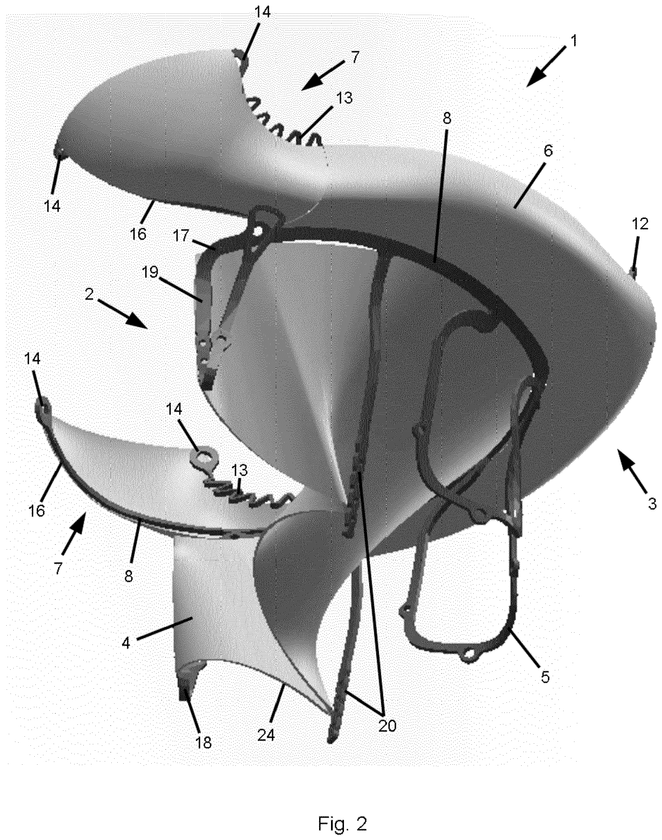

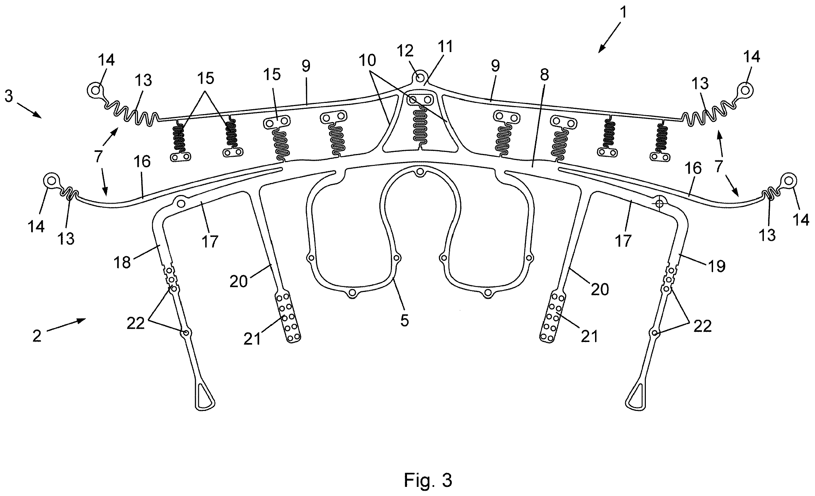

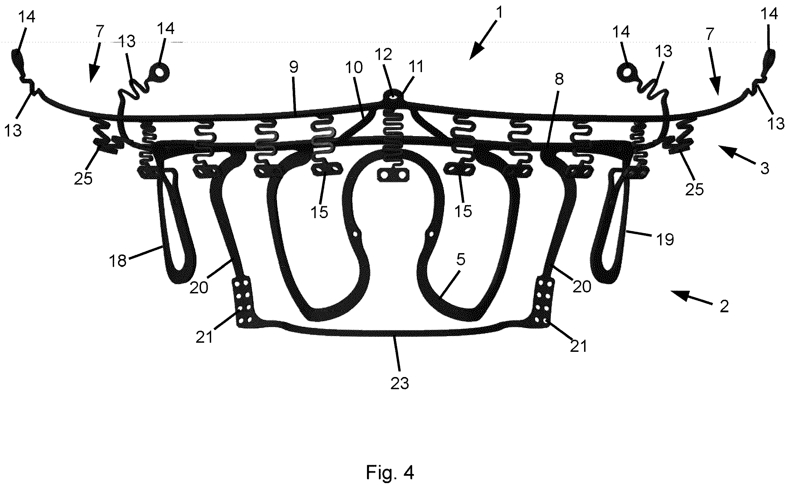

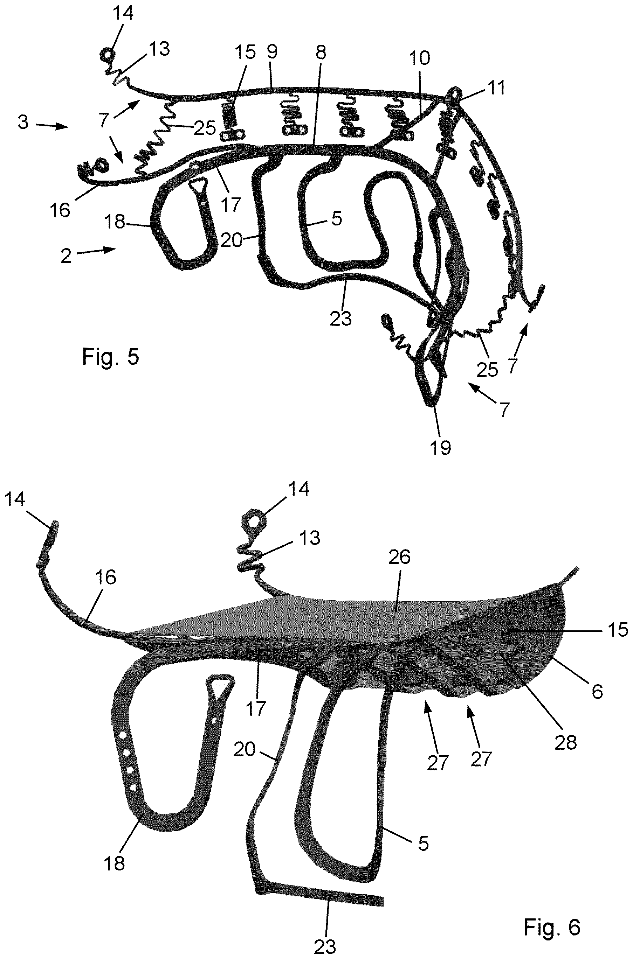

In the following, the invention will be explained in more detail with reference to exemplary embodiments shown in the figures. Therein, Fig. 1 shows a perspective front view of an implant according to the invention, Fig.2 shows a perspective rear view of the implant, Fig. 3 shows front view of the support frame of the implant of Fig. 1 and 2, Fig. 4 shows an alternative embodiment of the support frame, Fig. 5 shows a perspective view of the support frame of Fig. 4, and Fig. 6 shows a sectional perspective view of the support frame of Fig. 5 with the sealing skirt and a cover sheet being arranged on the upper frame section.

In Fig. 1 and Fig. 2, a mitral valve implant 1 is shown, which comprises a frame having a lower frame section 2 and an upper frame section 3. The lower frame section 2 supports an artificial leaflet 4 that is arranged and configured to cover the first native leaflet (not shown) and forms a coaptation plane for coaptation with the second native leaflet (not shown). The lower frame section 2 comprises or forms at least one central clasp 5 for attaching the lower frame section 2 to the first native leaflet. The upper frame section 3 supports a flexible sealing skirt 6 made of a two-dimensional element curved to rest against the atrial wall along the posterior annulus section, wherein the upper frame section 3 is made of a resilient material so that free end regions 7 of the upper frame section 3 are capable of being resiliently braced against the atrial wall and/or against the posterior annulus section.

The upper frame section 3 comprises an annulus bar 8 and an atrial bar 9, which are connected with each other by connecting bars 10 that are converging towards the center 11 of the upper frame section 3 or the atrial bar 9. At the center 11, an eyelet 12 is arranged as an attachment point for attaching the upper frame section 3 to the atrial wall. The free end regions 7 of the annulus bar and of the atrial bar each comprise an undulating section 13, which connects an attachment structure, such as an eyelet 14, located at a distal section of the free end regions 7 with a proximal section thereof. Further, a plurality of resilient spring elements 15 are extending form the annulus bar 8 and from the atrial bar 9 in an inward direction for pressing the sealing skirt 6 against the atrial wall.

The opposite end regions 7 of the annulus bar 8 are each split into an upper arm 16 and a lower arm 17, wherein the upper arms 16 are capable of being resiliently braced against the posterior annulus section and the lower arms 17 serve to fix the artificial leaflet 4 to the lower frame section 2.

The lower arms 17 can be considered to be part of the lower frame section 2 and support an anterolateral commissural clasp 18 for clasping the lower frame section 2 to the anterolateral commissure and a posteromedial commissural clasp 19 for clasping the lower frame section 2 to the posteromedial commissure. The anterolateral commissural clasp 18 and the posteromedial commissural clasp 19 are preferably integrally formed with lower arms 17. Further, the lower frame section 2 comprises leaflet support bars 20, to which the artificial leaflet 4 is attached. The leaflet support bars comprise attachment points, such as eyelets 21, for fixing the lower edge 24 of the artificial leaflet 4 to the support bars 20. At its lateral sides, the artificial leaflet 4 is fixed to the anterolateral commissural clasp 18 and the posteromedial commissural clasp 19, which also comprise respective attachment points, such as eyelets 22. In this way, the artificial leaflet 4 assumes the form of three scallops, thereby mimicking the shape of a native leaflet.

In Fig. 3, the elements of the implant 1 are denoted by the same reference numerals already used in Figs. 1 and 2. Fig. 3 shows the frame only, without the artificial leaflet 4 and the sealing skirt 6. Further, the anterolateral commissural clasp 18 and the posteromedial commissural clasp 19 are shown in a flat position before being bent to form a clasp, as shown in Figs. 1 and 2. It is noted that the frame may be manufactured, such as cut, from a single piece of metal.

Fig. 4 and 5 show an alternative embodiment, in which the leaflet support bars 20 are connected by a cross bar 23. Another difference to the embodiment shown in Figs. 1, 2 and 3 is the connecting wire 25 having an undulating shape, which connects the free end regions 7 of the annulus bar 8 and the atrial bar 9.

In the sectional perspective view shown in Fig. 6, the sealing skirt 6, together with the cover sheet 26, forms a pocket 28, into which blood can flow via the openings 27 that are formed in the sealing skirt 6.

## Claims

1. An implant for improving coaptation of an atrioventricular valve in a human heart, the atrioventricular valve having a first and a second native leaflet, an anterolateral and posteromedial commissure between the first and the second native leaflet, and an annulus adjacent an atrial wall of the heart comprising a posterior annulus section and an anterior annulus section, the implant (1) comprising a frame that comprises a lower frame section (2) that supports an artificial leaflet (4), the artificial leaflet (4) being arranged and configured to cover the first native leaflet and forms a coaptation plane for coaptation with the second native leaflet, wherein the lower frame section (2) comprises or forms at least one central clasp (5) for attaching the lower frame section (2) to the first native leaflet, the frame further comprising an upper frame section (3) that supports a flexible sealing skirt (6) made of a two-dimensional element curved to rest against the atrial wall along the posterior annulus section, wherein the upper frame section (3) is made of a resilient material so that free end regions (7) of the upper frame section (3) are capable of being resiliently braced against the atrial wall and/or against the posterior annulus section.

2. Implant according to claim 1, wherein the upper frame section (3) comprises an annulus bar (8) that extends in a curved manner along the posterior annulus section, wherein the opposite free end regions (7) of the annulus bar (8) are capable of being resiliently braced against the posterior annulus section and/or the atrial wall.

3. Implant according to claim 1 or 2, wherein the upper frame section (3) comprises an atrial bar (9) that extends in a curved manner along the atrial wall adjacent the posterior annulus section, wherein the opposite free end regions (7) of the atrial bar (9) are capable of being resiliently braced against the atrial wall.

4. Implant according to claim 1, 2 or 3, wherein the free end regions (7) comprise an undulating section (13), the undulating section (13) preferably connecting an attachment structure, such as an eyelet (14), located at a distal section of the free end regions (7) with a proximal section thereof.

5. Implant according to claim 3 or 4, wherein the free end regions (7) of the annulus bar (8) and the free end regions (7) of the atrial bar (9) are each connected with each other by means of a connecting wire (25), the connecting wire preferably having an undulating shape.

6. Implant according to any one of claims 2 to 5, wherein the free end regions (7) of the annulus bar (8) have a greater flexural rigidity in a first bending direction than in a second bending direction that extends orthogonal to the first bending direction, wherein limiting bending in the first bending direction is for preventing the implant from falling into the ventricle and bending in the second bending direction is for resiliently bracing the free end regions (7) against the posterior annulus section and/or the atrial wall.

7. Implant according to any one of claims 3 to 6, wherein a plurality of resilient spring elements (15) are extending form the annulus bar (8) and/or from the atrial bar (9) for pressing the sealing skirt against the atrial wall.

8. Implant according to any one of claims 1 to 7, wherein the upper frame section (3) carries a cover sheet (26) that, together with the sealing skirt (6), forms a pocket (28) for receiving retrograde blood flow, said blood flow acting upon the sealing skirt (6) to increase pressure of the sealing skirt (6) against the atrial wall.

9. Implant according to claim 8, wherein the sealing skirt (6) comprises one or more openings (27) for allowing retrograde blood flow to enter into the pocket (28).

10. Implant according to any one of claims 2 bis 9, wherein the central clasp (5) is connected to, or is integrally formed with, a central section of the annulus bar (8) arranged between the opposite free end regions (7) of the annulus bar (8).

11. Implant according to any one of claims 1 to 10, wherein the lower frame section (2) comprises an anterolateral commissural clasp (18) for clasping the lower frame section (2) to the anterolateral commissure and a posteromedial commissural clasp (19) for clasping the lower frame section (2) to the posteromedial commissure, the central clasp (5) being arranged between the anterolateral commissural clasp (18) and the posteromedial commissural clasp (19).

12. Implant according to claim 11, wherein the anterolateral commissural clasp (18) and the posteromedial commissural clasp (19) are connected to, or integrally formed with, the annulus bar (8), preferably the free end regions (7) of the annulus bar (8).

13. Implant according to any one of claims 1 to 12, wherein the lower frame section (2) comprises leaflet support bars (20), to which the artificial leaflet (4) is attached, said leaflet support bars (20) preferably extending in a ventricular direction from the annulus bar (8).

14. Implant according to claim 13, wherein a first one of said leaflet support bars (20) is arranged between the central clasp (5) and the anterolateral commissural clasp (18) and a second one of said leaflet support bars (20) is arranged between the central clasp (5) and the posteromedial commissural clasp (19).

15. Implant according to claim 13 or 41, wherein the leaflet support bars (20) are connected to, or integrally formed with, the annulus bar (8).

16. Implant according to claim 13, 14 or 15, wherein the leaflet support bars (20) are connected to each other, preferably at their free ends, by means of a cross bar (23).

17. Implant according to any one of claims 13 to 15, wherein the artificial leaflet (4) comprises an upper edge attached to the annulus bar (8) and a lower edge (24), opposite the upper edge, that comprises a plurality of attachment points arranged at a distance from each other, wherein the lower edge (24), at said attachment points, is fixed to the leaflet support bars (20), the anterolateral commissural clasp (18) and the posteromedial commissural clasp (19).

18. Implant according to any one of claims 13 to 17, wherein the opposite end regions (7) of the annulus bar (8) are each split into an upper arm (16) and a lower arm (17), wherein preferably the upper arms (16) are capable of being resiliently braced against the posterior annulus section and wherein preferably the anterolateral commissural clasp (18) and the posteromedial commissural clasp (19) and preferably the leaflet support bars (20) are connected to, or integrally formed with lower arms (17).
